# EUROPEAN PATENT SPECIFICATION

(11) **EP 1 570 079 B1**
(45) Date of publication and mention of the grant of the patent: **21.07.2010**
(21) Application number: 03782268.1
(22) Date of filing: 02.12.2003
(51) Int. Cl.: C12Q 1/68

(54) **MULTIPLEX ASSAY DETECTION OF PATHOGENIC ORGANISMS**
VERFAHREN ZUM NACHWEIS VON PATHOGENEN ORGANISMEN
METHODE POUR L'IDENTIFICATION DES ORGANISMES PATHOGENES

(30) Priority: 06.12.2002 EP 02027272; 04.04.2003 EP 03007458
(43) Date of publication of application: 07.09.2005
(73) Proprietor: Roche Diagnostics GmbH, 68305 Mannheim (DE); F. Hoffmann-La Roche AG, 4070 Basel (CH); Innogenetics N.V., 9052 Gent (BE)
(72) Inventor: HABERHAUSEN, Gerd, 82377 Penzberg (DE); EMRICH, Thomas, 82393 Iffeldorf (DE); SAGNER, Gregor, 82377 Penzberg (DE); MOCZKO, Martin, 82377 Penzberg (DE); SCHMITZ-AGHEGUIAN, Gudrun, 82347 Bernried (DE)
(86) International application number: PCT/EP2003/013530
(87) International publication number: WO 2004/053155

(56) References cited:
- EP-A- 0 452 596
- WO-A-01/48237
- WO-A-02/53771
- WO-A-96/00298
- US-A- 6 277 577
- US-A- 6 312 903
- US-A- 6 656 689
- ESPY ET AL: "Diagnosis of Herpes Simplex Virus Infections in the Clinical Laboratory by LightCycler PCR" JOURNAL OF CLINICAL MICROBIOLOGY, WASHINGTON, DC, US, vol. 38, no. 2, February 2000 (2000-02), pages 795-799, XP002175791 ISSN: 0095-1137 cited in the application
- SILVA DE D ET AL: "Rapid Genotyping and Quantification on the LightCycler with Hybridization Probes" BIOCHEMICA, MANNHEIM, DE, no. 2, 1998, pages 12-15, XP002175789 ISSN: 0946-1310
- BERNARD P S ET AL: "HOMOGENEOUS MULTIPLEX GENOTYPING OF HEMOCHROMATOSIS MUTATIONS WITH FLUORESCENT HYBRIDIZATION PROBES" AMERICAN JOURNAL OF PATHOLOGY, PHILADELPHIA, PA, US, vol. 153, no. 4, 1998, pages 1055-1061, XP000874040 ISSN: 0002-9440
- PIETILAE J ET AL: "Rapid Differentiation of Borrelia garinii from Borrelia afzelii and Borrelia burgdorferi Sensu Stricto by LightCycler Fluorescence Melting Curve Analysis of a PCR Product of the recA Gene" JOURNAL OF CLINICAL MICROBIOLOGY, WASHINGTON, DC, US, vol. 38, no. 7, July 2000 (2000-07), pages 2756-2759, XP002175790 ISSN: 0095-1137
- KLAUSEGGER A ET AL: "Gram Type-Specific Broad Range PCR Amplification for Rapid Detection of 62 Pathogenic Bacteria" JOURNAL OF CLINICAL MICROBIOLOGY, WASHINGTON, DC, US, vol. 37, no. 2, February 1999 (1999-02), pages 464-466, XP002176097 ISSN: 0095-1137
- ANTHONY R M ET AL: "Rapid Diagnosis of Bacteremia by Universal Amplification of 23S Ribosomal DNA Followed by Hybridization to an Oligonucleotide Array" JOURNAL OF CLINICAL MICROBIOLOGY, WASHINGTON, DC, US, vol. 38, no. 2, February 2000 (2000-02), pages 781-788, XP002176098 ISSN: 0095-1137
- RIRIE K M ET AL: "PRODUCT DIFFERENTIATION BY ANALYSIS OF DNA MELTING CURVES DURING THE POLYMERASE CHAIN REACTION" ANALYTICAL BIOCHEMISTRY, ACADEMIC PRESS, SAN DIEGO, CA, US, vol. 245, no. 2, 1997, pages 154-160, XP001015991 ISSN: 0003-2697

## Description

The invention relates to the technical field of detecting pathogenic microbial organisms. More specifically, the invention relates to the field of detecting an infection caused by a pathogenic organism in a clinical specimen by means of amplifying and detecting specific nucleic acid sequences from said pathogenic organism.

### Prior art background

Infection by pathogenic bacteria, particular if causing sepsis, is predominantly occurring and serious in intensive care units (ICU) of hospitals. The bacterium infecting the patient is in most cases unknown and cannot be determined from the symptoms. Each bacterium requires a different therapy using administration of a specific antibiotic. Presently, in routine diagnostics pathogenic bacteria, particularly Gram positive bacteria, are detected using a method including subjecting a sample of blood or other body fluid to culture the bacteria, if present. This culture is maintained at conditions favoring bacterial growth for about three days. During this time, the number of bacteria and thus their nucleic acids is increased. Thereafter, the culture medium is subjected to lysis. The lysis mixture is used as the sample for subsequent biochemical or immunological analyses. The overall method takes around four days minimum until clarity on any infection of the sample by pathogenic bacteria is reached. Infection by pathogenic bacteria is very serious for the infected person. Within the first day of infection a therapy, preferably by administration of an antibiotic suitable to specifically affect the particular infecting bacterium has to be started. Otherwise, the person is too heavily affected by the infection and may die before clarity on the infection is reached. On the other side, administration of several broad range antibiotics simultaneously to prevent systemic events has to be avoided to not weaken the patient. The present methods thus are not satisfactory for routine ICU diagnostics.

Identification of pathogenic organisms such as pathogenic bacteria or fungi by means of nucleic acid based hybridization using specific hybridization probes has been known in the art already for a long time. For example, EP 0 131 052 discloses methods and probes wherein ribosomal ribonucleic acid (rRNA) sequences of a certain species or a certain group of organisms are detected directly from culture media. Detection of ribosomal target sequences is especially useful due to the fact that these sequences are amplified in-vivo, resulting in high sensitivity of the respective assay.

An improvement of nucleic acid sequence based detection of pathogenic organisms was achieved upon availability of the PCR technology. For the detection of pathogenic fungi such as Candida and Aspergillus, for example, WO 97/07238 discloses a method using generic primers for amplifying all types of fungal ribosomal 18S rDNA sequences and subsequently hybridizing with fungi species specific probes.

As an alternative to the analysis of ribosomal gene sequences, non-coding but transcribed ribosomal spacer DNA sequences like the ITS-1 region located between the 16S and the 23S rRNA genes have been used for detection and identication of several pathogenic organisms (see, for example, EP 0 452 596).

In another context, the groups of Gram positive and Gram negative bacteria have been discriminated by means of a target-dependent amplification comparable to an allele specific amplification approach (Klausegger, A., et al., J. Clin. Microbiol. 37 (1999) 464-466). On the basis of their 16S r-DNA sequences, the species investigated by Klausegger et al. differ at a given position of the 16S rRNA gene in that all investigated Gram negative bacteria contain a G-residue at a certain nucleotide position whereas all investigated Gram positive bacteria always contain a C-residue at said nucleotide position. Consequently, usage of appropriate primers having either a discriminating complementary 3'-terminal C-residue or a complementary G-residue, respectively, results in DNA amplification of either Gram positive or Gram negative sequence origin.

Further progress was made upon availability of kinetic Real Time PCR. In this type of assay, formation of PCR products is monitored in each cycle of the PCR. The amplification is usually measured in thermocyclers having additional detection means for monitoring fluorescence signals during the amplification reaction. A typical example is Roche Diagnostics LightCycler™ (Cat. No. 2 0110468). In LightCycler™ as well as in other Real Time PCR instruments commercially available so far, amplification products are detected by means of fluorescently labeled hybridization probes which only emit fluorescence signals when they are bound to the target nucleic acid or in certain cases also by means of fluorescent dyes that bind to double-stranded DNA. A defined signal threshold is determined for all reactions to be analyzed and the number of cycles (Cp) required to reach this threshold value is determined for the target nucleic acid as well as for the reference nucleic acids such as a standard or housekeeping gene. The absolute or relative copy numbers of the target molecule can be determined on the basis of the Cp values obtained for the target nucleic acid and the reference nucleic acid (Roche Diagnostics LightCycler™ operator manual(Cat. No. 2 0110468)).

There exist different formats for the detection of amplified DNA:

### a) DNA binding dye format

Since the amount of double stranded amplification product usually exceeds the amount of nucleic acid originally present in the sample to be analyzed, double-stranded DNA specific dyes may be used, which upon excitation with an appropriate wavelength show enhanced fluorescence only if they are bound to double-stranded DNA. Such method is described in EP 0 512 334. Preferably, only those dyes are used, like for example SYBR® Green I, which do not affect the efficiency of the PCR reaction.

All other formats known in the art require the appropriate design of a fluorescently labeled hybridization probe which only emits fluorescence upon binding to its target nucleic acid.

### b) TaqMan™ probes

A single-stranded hybridization probe is labeled with two components. When the first component, the so-called fluorescer, is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3'-exonuclease activity of the polymerase, for example Taq Polymerase, during the elongation phase. As a result the excited fluorescent component and the quencher are spatially separated from one another and thus a fluorescence emission of the first component can be measured (EP B 0 543 942 and US 5,210,015).

### c) Molecular Beacons

These hybridization probes are also labeled with a first component and with a quencher, the labels preferably being located at different ends of an at least partially self-complementary probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

### d) FRET hybridization probes

The Fluorescence Resonance Energy Transfer (FRET) hybridization probe test format is especially useful for all kinds of homogenous hybridization assays (Matthews, J. A. and Kricka, L. J., Anal Biochem 169 (1988) 1-25). It is characterized by two single-stranded hybridization probes which are used simultaneously and are complementary to adjacent sites of the same strand of an (amplified) target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured only when both hybridization probes bind to adjacent positions of the target molecule to be detected.

When annealed to the target sequence, the hybridization probes must be located very close to each other, in a head to tail arrangement. Usually, the gap between the labeled 3' end of the first probe and the labeled 5' end or the second probe is as small as possible, i.e. 1-5 bases. This allows for a close vicinity of the FRET donor compound and the FRET acceptor compound, which is typically 10-100 Ångstrom. Particulars are well known and disclosed for example in EP 0 070 687.

Alternatively to monitoring the increase in fluorescence of the FRET acceptor component, it is also possible to monitor fluorescence decrease of the FRET donor component as a quantitative measurement of hybridization event.

In particular, the FRET hybridization probe format may be used in real time PCR, in order to detect the amplified target DNA. Among all detection formats known in the art of real time PCR, the FRET-hybridization probe format has been proven to be highly sensitive, exact and reliable (WO 97/46707; WO 97/46712; WO 97/46714). Yet, the design of appropriate FRET hybridization probe sequences may sometimes be limited by the special characteristics of the target nucleic acid sequence to be detected.

As an alternative to the usage of two FRET hybridization probes, it is also possible to use a fluorescent-labeled primer and only one labeled oligonucleotide probe (Bernard, P. S., et al., Anal. Biochem. 255 (1998) 101-7). In this regard, it may be chosen arbitrarily, whether the primer is labeled with the FRET donor or the FRET acceptor compound.

FRET hybridization probes (also called FRET-Hybprobes or FRET probes) can also be used for melting curve analysis (WO 97/46707; WO 97/46712; WO 97/46714). In such an assay, the target nucleic acid is amplified first in a typical PCR reaction with suitable amplification primers The hybridization probes may already be present during the amplification reaction or be added subsequently. After completion of the PCR-reaction, the temperature of the sample is constitutively increased. Fluorescence is detected as long as the hybridization probe is bound to the target DNA. At the melting temperature, the hybridization probe is released from their target, and the fluorescent signal is decreasing immediately down to the background level. This decrease is monitored with an appropriate fluorescence versus temperature-time plot such that the negative of a first derivative function can be calculated. The temperature value corresponding to the obtained maximum of such a function is then taken as the determined melting temperature of said pair of FRET hybridization probes.

Point mutations or polymorphisms within the target nucleic acid result in a less then 100% complementarity between the target nucleic acid and the FRET probes, thus resulting in a decreased melting temperature. This enables for a common detection of a pool of sequence variants by means of FRET-Hybprobe hybridization, whereas subsequently, different members of said pool may become discriminated by means of performing melting curve analysis. Instead of FRET hybridization probes, Molecular Beacons may alternatively be used for melting curve analysis.

Upon the availability of Real-Time PCR and homogenous Real-Time PCR melting curve analysis, discrimination of certain types of species or strains became possible using either double stranded DNA binding dyes such as SybrGreen™I, or, alternatively, specifically designed hybridization probes hybridizing to different but similar target sequences.

In the first case, melting temperature of the generated double stranded PCR product has to be determined. Yet, this method has only limited applications, because minor sequence variations only result in subtle melting temperature differences, cannot be monitored efficiently. A successful example has been disclosed by Woo,T.H., et al., J. Microbiol. Methods 35 (1999) 23-30, who performed amplification of Leptospira biflexa 16S rDNA sequences and subsequent melting curve analysis using SybrGreen™ I as a DNA binding dye in order to discriminate Leptospira biflexa strains from non-specific amplification products originating from the DNA of different Leptospira species.

Alternatively, hybridization probes may be used in such a way that the melting temperature of the probe/target nucleic acid hybrid is being determined. For example, Espy, M.J., et al., J. Clin. Microbiol. 38 (2000) 795-799 disclose diagnosis of Herpes simplex infections by means of amplifying a Herpes simplex sequence and subsequent analysis using FRET hybridization probes to discriminate between the HSV Type I and the HSV Type II genotype.

Furthermore, WO 01/48237 suggests in general the connection of amplification and temperature dependent hybridization in order to detect different pathogenic species. Yet, WO 01/48237 does not teach any methods or conditions which enable for an exclusive detection of pathogenic organisms, but to not detect any non pathogenic organism.

Silva et al., Biochemica Mannheim, DE, no. 2, 1998, p. 12-15, describe rapid genotyping and quantification on the LightCycler with hybridization probes.

Ririe et al., Analytical Biochemistry, vol. 245, no. 2, 1997, p. 154-160, disclose product differentiation by analysis of DNA melting curves during PCR.

WO 96/00298 A is concerned with the detection of pathogenic bacteria with a hybridization-based assay termed Line Probe Assay (LiPA). In this format, the probes are immobilized on a membrane strip and hybridize all at a common temperature.

WO 02/53771 discloses the identification of pathogenic E.coli, wherein real-time amplification methods are used. The use of melting curve analysis is also mentioned.

Even the presently used methods including in-vitro amplification of specific bacterial species and subsequent detection of said bacterium are not useful for cases where urgent diagnostics is needed, for example in ICU, because for each bacterium an amplification reaction and a detection reaction have to be performed. This requires that a large amount of sample volume, such as blood, has to be drawn from each patient. In ICU large sample volumes from severely infected patients are not available.

Thus, there is a need in the art to provide methods which are specifically applicable for detecting relevant pathogenic organisms of interest in a volume as small as possible. In particular, there is a need in the art for a method which enables for the detection of all relevant pathogenic Gram positive bacteria but at the same time does not detect similar non-pathogenic Gram positive bacteria. Particularly there is a need to determine which pathogenic genus and/or species is present in a sample.

### Brief Description of the invention

The problem disclosed above is resolved by methods disclosed and claimed in the present application.

In general, the present invention is directed to a method for identification of a pathogenic organism from a predetermined group of pathogens, comprising
a) isolating a clinical sample containing at least partially purified nucleic acid,
b) subjecting at least a first aliquot of said clinical specimen to at least one amplification and detection reaction in one reaction vessel comprising
   ba) an amplification step using at least a first set of amplification primers capable of amplifying a pre-selected 16s/23s or 18s/26s spacer region from several or all members of said predetermined group of pathogens,
   bb) a detection step using at least 2, 3 or multiple hybridization reagents, said reagents together being capable of specifically detecting a pre-selected 16s/23s or 18s/26s spacer region from all members of said group of pathogens, said detection step bb) comprising steps
      bba) monitoring hybridization of each of said hybridization reagents at a pre-selected temperature, said hybridization being indicative for at least the genus of said pathogen present in the sample, and
      bbb) monitoring temperature dependence of hybridization with said hybridization reagents and determining the melting temperature, said temperature dependence being indicative for at least the species of said pathogen,
c) determining if a hybridization signal is occurring in step bba) whether a pathogenic organism of a certain genus is present in said sample, and determining from the temperature dependence of step bbb), which pathogenic species of said genus is present in said sample.

### Detailed description of the invention

The present invention provides methods especially suitable for the diagnosis of an infection of a pathogenic organism . In this regard, if a patient is suspected to have an infection, the present invention provides a means to determine the species of said pathogenic organism. Moreover, the new methods according to the invention, especially when combined with Real Time PCR technology such as LightCycler™ system enable for a rapid diagnosis of an infectious agent causing sepsis, which is of outstanding importance in many clinical environments.

Thus, in general, the present invention is directed to a method for identification of a pathogenic organism from a predetermined group of pathogens, comprising
a) isolating a clinical sample containing at least partially purified nucleic acid,
b) subjecting at least a first aliquot of said clinical specimen to at least one amplification and detection reaction in one reaction vessel comprising
   ba) an amplification step using at least a first set of amplification primers capable of amplifying a pre-selected 16s/23s or 18s/26s spacer region from several or all members of said predetermined group of pathogens,
   bb) a detection step using at least 2, 3 or multiple hybridization reagents, said reagents together being capable of specifically detecting a pre-selected 16s/23s or 18s/26s spacer region from all members of said group of pathogens, said detection step bb) comprising steps
      bba) monitoring hybridization of each of said hybridization reagents at a pre-selected temperature, said hybridization being indicative for at least the genus of said pathogen present in the sample, and
      bbb) monitoring temperature dependence of hybridization with said hybridization reagents and determining, the melting temperature, said temperature dependence being indicative for at least the species of said pathogen,
c) determining if a hybridization signal is occurring in step bba) whether a pathogenic organism of a certain genus is present in said sample, and determining from the temperature dependence of step bbb), which pathogenic species of said genus is present in said sample.

Independently from the definition of the different steps of the method as claimed, it is understood that steps a), b), and c) are preferably performed subsequently one after the other. Additional steps may be performed before each step, between two steps, or after each step.

Starting material of the method of the invention is the clinical sample, which is suspected to contain a pathogenic organism. Examples of such clinical samples are whole blood, serum plasma and cerebrospinal fluid. Preferably the starting material is of human origin.

Step a) is always done first and can be performed according to any methods known in the art. In this context, a "clinical specimen" is understood as a specimen derived from a sample containing any kind of cellular or non cellular material which is obtainable from a patient, namely body fluid or even tissue. Preferably, the clinical specimen is derived from either whole blood, serum, plasma or cerebrospinal fluid.

Particularly, the nucleic acids are separated from proteins and sugars present in the original sample. Any purification methods known in the art may be used in the context of the present invention. Preferably, however, methods are applied which result in an essentially total purification of the total DNA to become analyzed. At least, purification needs to be performed to such an extend that without any further substantial dilution, nucleic acid sequences in aliquots of the sample can successfully be amplified in in-vitro amplification, such as the PCR. In purification, any compounds inhibiting polymerases are removed from the nucleic acids.

The amplification is done using an in-vitro method, preferably PCR (EP 201184). In the following, reference is made to PCR, but it is understood that other in-vitro amplification methods are suitable, too.

Step b) includes both heterogenous and homogenous embodiments. In the heterogenous embodiment, amplification step ba) is performed first, Subsequently, and optionally including supplementation with additional detection reagents, step bb) is performed.

In the homogenous embodiment, reagents for both the amplification and the detection step are preferably already added to the sample prior to the beginning of the amplification step ba). In some case, step ba) and step bba) may be performed in parallel, i. e. monitoring of hybridization is performed in real time, preferably during each or after each or at least after several of the performed thermocycles. In this case, said pre-selected temperature of step bba) is usually identical or very similar to the annealing temperature of the PCR thermocycling protocol.

Steps bba) and bbb) are usually done together in such a way that first, the hybridization event itself is monitored at a pre-selected temperature. Then, the temperature is constitutively increased in order to determine the temperature at which the probe/target hybrid is being resolved. In other words, a melting curve analysis is performed.

In case hybridization is monitored in real time already during the amplification reaction, performance of a melting curve analysis may be omitted, if it was not possible to find a hybridization signal at the pre-selected temperature.

In the context of the present invention, the specific terms used above are defined as follows:
The term "identification of pathogenic organism" is used to describe a method to determine whether a particular species or strain or isolate within a species contained in the predetermined group or subgroup of pathogenic organisms is present in the sample. The output or result of the identification is the name of the species. This will allow subsequently for an appropriate selection of a selective antibiotic therapy.

The term "predetermined group of pathogenic organisms" is used to describe a predefined group of pathogenic organisms consisting of pathogenic organisms which are of interest for a particular task. Preferably, a predetermined group of pathogenic organisms may be the group of all pathogenic organisms, which are clinically relevant under given clinical circumstances, and the respective genomic sequences of which to become amplified are substantially known in the art.

For example, such a predetermined group may comprise the clinically relevant members of two or more genera. Alternatively, all known clinically relevant members of a certain genus may constitute such a predetermined group. Alternatively, all known clinically relevant members or strains or isolates of a certain species may constitute such a predetermined group. The predetermined group can also contain taxonomic sub-groups of different genera, mixed with strains from other genera or species.

The term "specific" is used to describe the characteristic of a subject (for example in methods, steps or reagents) that the subject is directed to only a particular and defined result. For example, a method for the detection of a particular species is considered to be specific, if only this species, but not other species are detected. Specific hybridization of a probe with a target is a hybridization which only occurs between the probe and the target, but not with other nucleic acids. In other words, the present invention allows for a selective detection of all pathogenic members of a genus or a species, whereas other known, non-pathogenic members of said genus or said species are definitely not detected.

In this context, the overall method of the present invention is preferably substantially specific regarding the identification of the organism. Organisms not being a member of the predetermined group or subgroup are preferably not identified, because the steps performed with the reagents are adjusted to not detect organisms not belonging to that group. This does not necessarily mean that each single step of the overall method is specific, while this is possible. For example, if the amplification primers are chosen to be not specific for the organism to be identified (in that those primers can be used to amplify nucleic acids of organisms not to be identified), step ba) can be non-specific. Specificity of the overall method can then be achieved by selecting one or more of the parts of the detection step to be specific, for example step bbb). By way of example, the present invention covers embodiments, where step bba) results in a positive signal, due to hybridization of the hybridization reagent with a nucleic acid of a member of the predetermined group and in addition to non-target nucleic acids in the specimen or/and amplified in step ba), but in step bbb) monitoring temperature dependence only proves the identity of the species of the member of the predetermined group, independently of the identity of the non-target nucleic acids.

As a main advantage compared to methods known in the art, the present invention allows for the first time a selective identification of the pathogenic members of a bacterial genus or a species, whereas other known, non-pathogenic members of said genus or said species are not identified.

It is important to note that in case a clinical specimen to be analyzed contains a new, previously unknown strain with a new, slightly differing target sequence, the new method according to the invention may occasionally lead to either a false positive or a false negative result. In case an unknown non-pathogenic organism is amplified and detected, the result of step bba) will become false positive. Yet, in this case, the false positive result may become detected during step bbb) of the claimed method, i. e. the monitoring of temperature dependence of hybridization. In this case, comparing the temperature dependency of step bbb) to the temperature dependence of the known pathogenic organisms will show that the organism does not belong to the species of the predetermined group, i.e. the result of step c) will be negative. In case an unknown pathogenic organism is either not amplified or not detected, the result is false negative. Yet according to all studies on clinical isolates performed by the inventors so far, these cases are very rare and, moreover, are not avoided by any other comparable method known in the art.

The term "predetermined sub-group of pathogenic organisms" is used to describe a part or the whole of the predetermined group of pathogenic organisms. Preferably, the predetermined subgroup is only a small part of all pathogenic organisms, more preferably the pathogens predominantly occurring in hospitals. The members of the group and the subgroup can be chosen from one genus, but can also be chosen from different genera. In case the predetermined group is represented by all pathogenic and clinically relevant members of a genus, a respective sub-group may contain all respective pathogenic members of a certain species. Similarly, if said predetermined group consists of all clinically relevant members of a certain species, then the sub-group may consist of all members of a specific strain.

The term "amplification and detection reaction" shall mean any kind of in-vitro method comprising one or more "amplification steps" in order to amplify one or more target sequences from a pool of nucleic acid sequences and one or more "detection steps" in order to unravel the identity of the amplified product.

The term "amplification step" shall mean a reaction or a series of reactions that amplifies, a target sequence - if present in the clinical specimen - by means of using forward and reverse primers for a nucleic acid amplification reaction from a specimen containing DNA. The specificity of said amplification step depends upon the group selected and is governed by the specificity of the primers used. Preferably, amplification is obtained by means of a polymerase chain reaction (PCR) using a thermostable DNA polymerase. In one embodiment, the amplification is specific for bacteria generally; i.e. viruses are not amplified in substantive amounts.

Preferably, the amplification step is specific for the members of the subgroup. In a preferred embodiment, amplification is specific to the genus to which the species to be identified belong.

The term "detection step" shall mean that the amplification product is detected by means of hybridization with an appropriate hybridization reagent, including monitoring temperature dependence of hybridization of said reagent to the target nucleic acid. Amplification and detection do not necessarily be separated and performed serially, but can be performed simultaneously.

The term "pre-selected nucleic acid sequence region" shall mean a distinct target nucleic acid region present in the DNA of all organisms intended to be amplified and detected. Depending on the embodiment, there may exist some sequence variations between the sequences of different organisms. In other words, it is always the same gene or the same homologous sequence of each organism, which is amplified.

It has been proven to be advantageous, if the preselected nucleic acid sequence region is present in multiple copies in the genome of the target organisms which shall become detected.

Excellent pre-selected nucleic acid sequences regions are the transcribed spacer regions of rDNA gene clusters.

In this regard, it has been proven by the inventors to be particular advantageous, if the pre-selected nucleic acid sequence region is corresponding to the internal transcribed spacer region I, which is always located in between a copy of the 16S rRNA gene and a copy of the 23S rRNA gene (ITS-1 region). This region contains evolutionary conserved as well as hypervariable sequences, which allow for a flexible design of both, genus and species specific primers and probes In eucaryotes such as pathogenic fungi, there exist an analogous transcribed spacer region between the 18s rDNA and the 26s rDNA..

More preferably, the preselected nucleic acid sequence region contains at least a part of 20, even more preferred more than 40 contiguous nucleobases from the 16S/23S or 18s/26s spacer region of the organisms to be amplified. This region contains evolutionary conserved as well as hypervariable sequences, which allow for a flexible design of both, genus and species specific primers and probe. The region is contained within the region defined by the primer binding sites on the nucleic acid.

The term "pathogenic" means that the bacterium may affect the health status of a human being, if that human being is infected by that bacterium. In particular, the invention is directed to the identification of bacteria and fungi causing sepsis.

The term "set of amplification primers" shall mean at least two (extendable) oligonucleotides or oligonucleotide derivatives, i. e. at least one (forward) primer binding to a first strand of the target nucleic acid and at least a second (reverse) primer binding to the opposite strand of the target nucleic acid sequence to be amplified. Moreover, the positioning of the primers is designed in such a way that template dependent extension of each primer generates an extension product which itself comprises a primer binding site to which the other primer can hybridize.

In most cases, it is sufficient to use a pair of two amplification primers consisting of one forward primer and one reverse primer. Yet, in some cases there may exist some minor sequence variants in the sequences of the primer binding sites of different sequences of different pathogens to be identified. Thus it may be impossible to amplify the sequences of all members by just using one forward and one reverse primer. For those cases, a set of amplification primers may consist of 1, 2, or more forward primers and/or 1, 2 or more reverse primers, which are similar and bind to homologous sequences, but differ from each other by one, two, three or several mononucleotide-, dinucleotide- or trinucleotide exchanges, deletions, or additions.

Moreover, it is also within the scope of the invention, if two, three or multiple sets of amplification primers capable of amplifying different pre-selected nucleic acid sequence regions are used. In this case, said different pre-selected nucleic acid sequences may not necessarily be related to each other in sequence. Yet, it is also within the scope of the present invention, if the different pre-selected nucleic acid sequence regions are at least partially or almost completely overlapping.

In general, the design of amplification primers is performed on the basis of available sequence information with regard to the pre-selected target nucleic acid sequence regions of the pathogenic bacteria to be amplified as well as with regard to the homologous sequences of those organisms, which shall not be amplified. More precisely, the set or sets of amplification primers are selected in such a way that there is a maximum sequence complementarity with respect to all target nucleic acid sequences of the selected predetermined group of pathogenic organisms, and, on the other hand, a minimum sequence complementarity with respect to nucleic acid sequences of all other non-selected organisms, i.e. those not belonging to the predetermined group or not being pathogenic.

The term "hybridization reagent" is used to describe a reagent capable of hybridizing to products of amplification of step bb) within the preselected nucleic acid sequence region; i.e. on at least one strand of the amplicon(s). The reagent can comprise one or more probes, which preferably are single stranded or are made single stranded prior to hybridization. Preferably the reagent is a single stranded nucleic acid hybridization probe system, comprising usually one or two nucleic acids which are capable of hybridizing to one strand of the double stranded amplified target nucleic acid. Depending on the type of detection format, it is in most cases advantageous if the hybridization reagent is appropriately labeled with a detectable entity, such that by detection of said label, the amplicon/hybridization-reagent hybrid can be detected.

In a preferred embodiment, the hybridization reagent is labeled with a fluorescent entity such that hybridization can be detected in commercially available Real Time PCR instruments. Reagents useful for this are disclosed in the documents mentioned above describing the different formats for the detection of amplified DNA.

The hybridization reagent in a simple case may be an oligonucleotide probe. For example, the Molecular Beacon Format (US 5,118,801) may be applied. Alternatively, it is also possible to use appropriate single labeled oligonucleotides (WO 02/14555).

More preferably, the hybridization reagent is composed of two adjacently hybridizing oligonucleotides, appropriately labelled such that together they can act according to the FRET-Hybprobe detection format as disclosed above (WO 97/46707; WO 97/46712; WO 97/46714).

More preferably, the hybridization reagent is composed of two adjacently hybridizing oligonucleotides, appropriately labeled such that together they can act according to the FRET-Hybprobe detection format as disclosed above (WO 97/46707; WO 97/46712; WO 97/46714). In many cases, it is sufficient if the hybridization reagent consists of a single oligonucleotide or in case of the FRET hybprobe format, of a pair of oligonucleotides acting together as a donor probe and an acceptor probe. Yet, in other cases there may exist many other sequence variants in the target sequences of different Fungal pathogenic bacteria, which need to be detected. Thus it may be impossible to detect the sequences of all members by just using one oligonucleotide as a probe or just using just one pair of FRET oligonucleotide hybridization probes.

For those cases, a hybridization reagent may consist of 1, 2 or more hybridization probes, which are similar and bind to homologous sequences, but differ from each other by 1, 2, 3 or more mononucleotide-, dinucleotide- or trinucleotide exchanges, deletions or additions. In case of the hybridization reagent being a pair of FRET hybridization probes, said hybridization reagent may consist of 1, 2, 3, or more FRET donor oligonucleotide probes and/or 1, 2, 3, or more acceptor oligonucleotide probes. In this case all donor probes may be similar, but differ from each other by mononucleotide-, dinucleotide- or trinucleotide exchanges, deletions or additions. As well, all acceptor probes are similar, differ from each other by mononucleotide-, dinucleotide- or trinucleotide exchanges, deletions or additions.

It is explicitly emphasized that in addition to embodiments of using one hybridization reagent, the present invention is also directed to embodiments wherein 2, 3, 4 or multiple hybridization reagents, are used. In those cases, a discrimination of different hybridization signals may be obtained if each of the hybridization reagents is labeled with a different detectable entity, for example, a differently fluorescing compound. The hybridization target sequences may not necessarily be related to each other. Yet, it is also within the scope of the present invention, if said target sequences sequences are at least partially or almost completely overlapping.

In case of using multiple hybridization reagents on the basis of the FRET hybprobe format, only a single excitation source may be available. Then, it is preferable to have a common FRET donor dye and different acceptor dyes for each pair of FRET hybridization probes. For example, fluorescein or fluorescein derivatives may be used as a general FRET donor dye, being capable of interacting with a large number of FRET acceptor dyes such as LC-Red 640 (Roche Applied Science), LC-Red 705 (Roche Applied Science), Cy 5 or Cy 5.5 (Amersham). A specific embodiment is directed to methods on the basis of the FRET hybridization probe format, wherein each of said multiple hybridization reagents comprises the identical FRET donor probe with an identical sequence and an identical label, such as e.g. fluorescein, and wherein each of said hybridization probes comprises a different acceptor probe, each labeled with a different fluorophor.

Furthermore, in this context, the term "FRET pair" is defined as a pair of fluorescent labels that act together to create a FRET process, i.e. it consists of a FRET donor moiety and a FRET acceptor moiety.

Similar to the design of a set of amplification primers, the design of a hybridization reagent or multiple hybridization reagents is also performed on the basis of all available sequence information with regard to the pre-selected target nucleic acid sequences to be amplified and detected as well as with regard to the homologous sequences of those pathogenic organisms, which shall not be detected. More precisely, the sequences of the hybridization reagent(s) are selected in such a way that there is maximum sequence complementarity with respect to all target nucleic acid sequences of the selected predetermined group of pathogenic organisms, and, on the other hand, a minimum sequence complementarity with respect to all homologous nucleic acid sequences of all other non selected organisms.

In addition, the design of the hybridization reagent needs to take into account that a discrimination of several target sequence variations is possible on the basis of monitoring temperature dependence of hybridization. The present invention requires different melting temperatures for hybridization of a hybridization reagent to different target sequence variants originating from different pathogenic organisms. Thus, the sequences of a hybridization reagent according to the invention are designed in such a way that the calculated melting temperatures (calculated by methods known in the art) of the different hybridization reagent/target sequence hybrids differ from each other by at least 2°C, preferably by 4°C and most preferably by at least 6°C.

Summarizing, the invention provides a possibility for the design of genus specific hybridization reagents, characterized in that they detect all pathogenic members of a certain genus belonging to a certain predetermined group. Alternatively, species or strain specific hybridization reagents may be designed, which allow for a detection of all pathogenic strains of a certain species belonging to a certain predetermined group.

The term "monitoring temperature dependence of hybridization" shall mean that the melting temperature is determined, at which the probe is dissociating from the hybridization complex formed with the target nucleic acid. Precisely, the melting temperature (Tm) of a hybrid is defined as the temperature at which the maximum of a first derivative of the hybridization versus temperature signal plot is reached. The Tm is a characteristic of a hybrid depending upon complementarity of the strands.

In this context, it is important to note that the melting temperature (Tm) of a hybrid is depending on several factors independent from the actual the target nucleic acid sequence itself, such as salt concentration, length and GC content of the probe. Yet in addition, the melting temperature strongly depends on the number of mismatches, i. e. degree of complementarity between the probe and the target sequence. As a consequence, different melting temperatures are obtained for different variants of target sequences which may be found in different strains of a distinct species or different species of a distinct genus. Thus, using an appropriate probe design, one type of hybridization reagent may be used for both the detection of all members of a pre-selected group of organisms and the discrimination of said members by means of monitoring temperature dependence of hybridization.

It has been proven to be particular advantageous, if the temperature dependence of hybridization is determined by means of a melting curve analysis. More precisely, subsequent to the amplification reaction, the PCR product (in the presence of the hybridization reagent) is denatured at a first temperature, for example 90-100°C, and subsequently cooled down to a second temperature, said second temperature being below or close to the annealing temperature of the PCR protocol. Then, the temperature is increased at rates between 0,01-10°C/s, preferably 0,1-2°C/s, and most preferably 0,1-0,5°C/s.

The term "indicative for at least the genus" shall mean that if a hybridization signal is occurring in step bba), then it can be concluded for step c) that a pathogenic organism of a certain genus, respectively, may be present in the sample collected from the patient. Depending on the design (for example, the sequence) of the set or sets of amplification primers, and moreover, depending on the design of the hybridization reagent(s), a hybridization signal obtained from the signal of a distinct hybridization reagent may even be indicative for the species of the pathogenic organism to be detected. Preferably, the hybridization signal determined from step bba) is unambiguously indicative for the presence of one of the members of the predetermined group, but does not necessarily directly allow to know which of the members is present.

The term "indicative for at least the species" shall mean that on the basis of the result of monitoring temperature dependence of hybridization as monitored in step bbb) it can unambigously be concluded for step c) which pathogenic species is present in the clinical sample. Due to the design of the set or sets of amplification primers and the design of the hybridization reagent(s), the data obtained from monitoring the temperature dependence of hybridization are indicative for the identity of a species or even a certain strain of the respective pathogenic organism. Even if the specimen may contain non-target sequences similar or identical to the target sequence, they will not be amplified as the primers are not suitable for amplifying the non-target region.

Summarizing, the invention provides a method for the identification of pathogenic organisms, wherein in a first amplification step using one or several appropriate sets of primer pairs sequences of all pathogenic organisms of interest are amplified and subsequently detected by appropriate hybridization reagents. Due to an appropriate primer and probe design, sequences of non-pathogenic microbial organisms are either not amplified or not detected, or amplified, but not detected. In case hybridization with a distinct hybridization reagent occurs, it is indicative for at least the the genus of the infectious agent.

Subsequently, monitoring temperature dependent fluorescence is performed, for example in the form of subjecting the sample to a continuous increase of temperature and determining the temperature at which melting between the target nucleic acid and the hybridization reagent occurs. The monitored melting temperature is then indicative for at least the species or even the sub-species or strain of the respective pathogen that has been present in the original specimen.

In this context the melting temperature (Tm) of a hybrid is defined as usual in the art, i.e. the temperature at which the maximum of a first derivative of a hybridization versus temperature plot occures. The Tm is a characteristic of a hybrid depending upon complementarity of the strands.

In order to achieve a sufficient sensitivity for detecting an infection, step a of the inventive method usually includes an at least partial purification of the nucleic acid from the original sample. The nucleic acid to be isolated can either RNA or DNA or a mixture thereof. As the most preferably embodiment of the invention uses bacterial DNA for the final identification of the pathogenic organism, it is not required that the clinical specimen contains RNA. Therefore, it is not necessary to partially or even completely isolate exclusively RNA from the clinical sample. The isolation of DNA from the clinical sample should be as sufficient and complete as necessary to receive a signal with a positive control.

The result of step a) usually is a fluid comprising nucleic acids from the original clinical sample and in addition reagents added during the isolation step, like buffers. In step b), the clinical specimen or a part thereof are subjected to one or more amplification reactions. The amplification and detection reactions can comprise one or more steps. Both, amplification and detection reactions are known in the art. The amplification is done using an in-vitro method, preferably PCR (EP 0 201 184). In the following, reference is made to PCR, but it is understood that other in-vitro methods are suitable, too. The result of the amplification reaction is the production of a large number of extension products of said primers, predominantly having a sequence reaching from the 5'-terminal position of one primer to the 5'-terminal position of the other primer. Those nucleic acids produced are usually called "amplicons".

In order to avoid false negative results due to inhibitory residual components which may be present in the clinical specimen and for quantification purposes, it has been proven to be particular advantageous, if an internal control template is added. Usually, said control template comprises a known sequence with primer binding sites complementary to at least one set of amplification primers used for amplification of the target nucleic sequences to be detected. Consequently, said set of amplification primers is also capable of priming amplification of said selected sequence of the control template. Details of using internal standards, particularly for quantification, are disclosed in EP 0 497 784.

It has proven to be advantageous if the method according to the invention is performed using an aliquot of a clinical specimen which has a volume of between 10 and 100 µl. Thus, the method of the present invention can be performed with a sufficient sensitivity, if only a small amount of a clinical sample such as whole blood or serum is available.

Yet, eventually, the results of the inventive method or any other method known in the art may become affected by a high background of human genomic DNA present in a clinical specimen. If this is the case, a pre-amplification step according to WO 01/94634 may be performed, characterized in that selectively non-human DNA sequences are selectively amplified.

Referring to step b), as already indicated above, the present invention is directed to embodiments, wherein at least a first and a second, or, alternatively, a first, a second, and a third, or even a first, a second, a third and a fourth hybridization reagent are used in order to detect a broad range of pathogenic organisms. Preferably, each of the hybridization reagents is carrying a different label, preferentially a fluorescent label. Thus, according to the invention, all of the hybridization reagents may already be present during step ba) in a kind of multiplex approach, allowing detection of a multitude of pathogenic bacteria within one reaction vessel.

In order to avoid false negative results due to inhibitory residual components which may be present in the clinical specimen, it has been proven to be particular advantageous, if an internal control template is added. Usually, said control template comprises a selected sequence with primer binding sites complementary to at least one set of amplification primers used for amplification of the target nucleic sequences to become detected. Consequently, said set of amplification primers is also capable of priming amplification of said selected sequence of the control template.

For step b) there are both heterogenous and homogenous embodiments possible. In the heterogenous embodiment, the reagents necessary for step ba) are added first and amplification step ba) is performed first. Subsequently, and optionally including supplementation with additional detection reagents, step bb) is performed.

In some cases it may be advantageous to use the heterogenous formate, characterized in that subsequent to the amplification step ba), the reaction mixture is divided into at least two, three, four or several sub-aliquots. Step bb) is then performed with at least the same number of different hybridization reagents, each in a different reaction vessel.

In the homogenous embodiment which is highly preferred over the heterogenous embodiment, reagents for both the amplification and the detection step are added to the specimen prior to the beginning of amplification step ba). In some case, step ba) and step bba) may be performed in parallel, i. e. monitoring of hybridization is performed during amplification, in case of PCR during or after each or at least after several of the performed thermocycles. In this case, preferably said pre-selected temperature of step bba) is usually identical or very similar to the annealing temperature of the PCR thermocycling protocol. The annealing temperature is the temperature at which the hybridization reagent (for example the probe) hybridizes to its target (i.e. the nucleic acid to be amplified, or/and the amplicon formed in earlier extension reactions). In order to achieve sufficient specificity of the hybridization (i.e. to predominantly detect sequences of the target), the annealing temperature is selected such that the probes predominantly anneal/hybridize to the target nucleic acid(s), but not to nucleic acids of organisms not to be identified. Means to influence the selectivity of hybridization of probes to nucleic acids are widely known (length, GC-content and degree of complementarity).

According to the present invention, the amplification step ba) and the detection step bb) are preferably carried out subsequently in a homogenous assay format, characterized in that the one or more hybridization reagents are already present within the reaction mixture during the amplification step. In other words, amplification step ba) and detection step bb) are carried out within the same reaction vessel and without addition of further reagents between the steps.

Step bba) is a step wherein the signal of hybridized hybridization reagent is measured and will be done as in conventional homogenous nucleic acid hybridization, particularly as outlined above for the different methods on LightCycler.

Steps bba) and bbb) are preferably done together in such a way that first, the hybridization event itself is monitored at a pre-selected first temperature. Then, the temperature is continuously increased to at least the temperature at which the hybrid containing the hybridization reagent is resolved. In other words, a melting curve analysis for the hybrid is performed.

The monitoring step bbb) preferably is performed once a sufficient number of cycles Cp were performed to amplify any target sequence present up to a level to be detectable via temperature dependence. That is usually coinciding with the amount of nucleic acid detectable through probe hybridization, i.e. as soon as there is a clearly positive signal in step bba) measured, step bbb) can be performed. Cp is preferably between 20 and 50.

In case hybridization is monitored during the amplification reaction (which is also called "Real Time"), performance of a melting curve analysis may be omitted, as long as it is not possible to find a hybridization signal at the pre-selected temperature.

Step c) contains interpreting the results which have been obtained during steps bb). This may be done manually by interpretation of the results obtained. Preferably, the results are analyzed using computer programs which generate an output that clearly indicates whether and which Gram positive bacterium was present in the sample and thus may have caused the infection.

The interpretation is based on the correlation of the signals obtained in steps bba) and bbb) with values known from positive controls. The use of positive controls is also generally known from the prior art. A positive control is a nucleic acid which is known to be present in the specimen or in an artificially prepared control specimen. For example, the specificity of hybridization of the probe at the preselected temperature is used to determine whether any of the nucleic acids that could have been amplified by the primers in step ba) and which belong to said predetermined group of pathogenic organisms are present in the specimen. A positive signal (over a negative control) is indicative of the presence of a species belonging to said genus (step bba), even if the identity of said species is not determined from said step bba).

The temperature dependence of hybridization is used for identification of the species present in the specimen. By this step, it is determined to which species of said genus said organism, the general presence of which is determined from step bba, belongs. For example, its melting temperature is indicative of a nucleic acid of a particular species. Therefore, the presence of a predetermined species in the actual sample can be verified by the occurrence of a change of signal when the melting temperature of the hybrid of the target with the hybridization reagent is reached.

Useful for the present invention can be a composition comprising at least a first set of amplification primers and at least two, three or multiple hybridization reagents, characterized in that
- said at least first set of amplification primer is capable of amplifying a pre-selected nucleic acid sequence region from several or all members of a predetermined group of pathogens, and
- said at least 2, 3 or multiple hybridization reagents together are being capable of specifically detecting a pre-selected nucleic acid sequence region from all members of a predetermined group of pathogens, wherein
- hybridization of each of said hybridization reagents at a pre-selected temperature is indicative for at least the genus of a pathogen present in the sample, and
- the temperature dependence is indicative for at least the species of said pathogen.

In addition, such a composition may comprise one or several or preferably all compounds and reagents selected from the following list:
- Buffer, applicable for a polymerase chain reaction
- Desoxynucleoside triphosphates
- Template dependent DNA polymerase, preferably thermostable,

Such a composition may further comprise an at least partially purified nucleic acid, which may e.g. be originated from a clinical specimen and become subjected to any of the methods according to the present invention.

Also useful for the method according to the invention can be a kit comprising at least a first set of amplification primers and at least two, three or multiple hybridization reagents, characterized in that
- said at least first set of amplification primer is capable of amplifying a pre-selected nucleic acid sequence region from several or all members of a predetermined group of pathogens, and
- said at least 2, 3 or multiple hybridization reagents together are being capable of specifically detecting a pre-selected nucleic acid sequence region from all members of a predetermined group of pathogens, wherein
- hybridization of each of said hybridization reagents at a pre-selected temperature is indicative for at least the genus of a pathogen present in the sample, and
- the temperature dependence of said hybridization is indicative for at least the species of said pathogen.

In addition, such a kit may comprise one or several other compounds and reagents selected from the following list:
- Buffer, applicable for a polymerase chain reaction
- Desoxynucleoside triphosphates
- Template dependent DNA polymerase, preferably thermostable,
each separately or in combination. Such a kit may further comprise an at least partially purified nucleic acid, which may e.g. be originated from a clinical specimen and become subjected to any of the methods according to the present invention. Such a hit may also comprise an internal control DNA, which can be amplified and detected using the same primers and probes as used for the detection of any target nucleic acid.

Each of the components disclosed above may be stored in a single storage vessel. Yet, any combination of components for storage within the same vessel is possible as well.

Furthermore, such a kit may also comprise software tools such as compact discs carrying computer programs for qualitative or quantitative analysis of the data obtained by the claimed method.

The invention differs from prior art bacterial assays in that the known assays only provide information on one particular species or strain (conventional method for the detection of the presence of a particular organism in a sample), the reagents described supra are suitable for and used for the potential identification of two or more species, while in parallel indicating whether any of them (irrespective which organism) is present.

As indicated above, the combination of monitoring amplification dependent signals being indicative for at least the genus of a pathogen and monitoring temperature dependence of hybridization allows for detecting a simultanous detection and identification of a broad range of pathogenic organisms of interest.

In one particular embodiment, the present invention enables for the detection and identification of a predetermined group of pathogenic gram positive bacteria, said group comprising Staphylococcus aureus, Staphylococcus epidermidis, Staphylococcus haemolyticus, Streptococcus pneumoniae, Streptococcus agalactine, Streptococcus pyogenes, Enterococcus faecium, Enterococcus faecalis.

In a second particular embodiment, the present invention enables for the detection and identification of a predetermined group of pathogenic gram negative bacteria, said group comprising Escherichia coli, Klebsiella pneumoniae, Serratia marcescens, Enterobacter cloacae, Enterobacter aerogenes, Proteus mirabilis, Pseudomonas aeruginosa, Acinetobacter baumanii, Stenotrophomonas maltophilia.

In a third particular embodiment, the present invention enables for the detection and identification of a predetermined group of pathogenic fungi, said group comprising Candida albicans, Candida tropicalis, Candida parapsilosis, Candida crusei, Candida glabrata, Aspergillus fumigatus.

Preferably, said groups mentioned above do not comprise any other species.

It is also within the scope of the present invention, if after step a) of the inventive method, a first and a second aliquot are each subjected to an amplification and detection reaction according to steps b) and c) independently from each other in two different reaction vessels.

For example, the first aliquot can be used for the detection and identification of pathogenic gram positive bacteria such as Staphylococcus aureus, Staphylococcus epidermidis, Staphylococcus haemolyticus, Streptococcus pneumoniae, Streptococcus agalactine, Steptococcus pyogenes, Enterococcus faecium, and Enterococcus faecalis. The second aliquot can be used for the detection and identification of pathogenic gram negative bacteria such as Escherichia coli, Klebsiella pneumoniae, Serratia marcescens, Enterobacter cloacae, Enterobacter aerogenes, Proteus mirabilis, Pseudomonas aeruginosa, Acinetobacter baumanii, and Stenotrophomonas maltophilia.

In addition, it is within the scope of the present invention, if after step a) of the inventive method, a first and a second and a third aliquot are each subjected to an amplification and detection reaction according to steps b) and c) independently from each other in three different reaction vessels. In case the first and the second aliquots are used in order to detect and identify all relevant pathogenic gram positive and gram negative bacteria, repsectively, the third aliquot is used to detect and identify fungal pathogens such as Candida albicans, Candida tropicalis, Candida parapsilosis, Candida crusei, Candida glabrata, Aspergillus fumigatus.

It is convenient and time saving to run the analysis of the two or three different aliquots of clinical specimen mentioned above are run in parallel the same instrument. Therefore , it is highly preferred, if the amplification steps of the different amplification and detection reactions are performed with the same thermocycling profile. In other words, each amplification should be performed using identical annealing, elongation, and denaturation time and temperature parameters.

Furthermore, a kit useful for carrying out the method according to the invention may be composed of a combination of reagents useful for parallel analysis of a clinical specimen in different aliquots. Such a kit comprises at a first, a second and optionally a third set of amplification primers and at a first, a second and optionally a third set of at least two, three or multiple hybridization reagents, each characterized in that
- each of said sets of amplification primer is capable of amplifying a pre-selected nucleic acid sequence region from several or all members of a predetermined group of pathogens, and
- each of said sets of at least 2, 3 or multiple hybridization reagents together are being capable of specifically detecting a pre-selected nucleic acid sequence region from all members of a predetermined group of pathogens, wherein
- hybridization of each of said hybridization reagents at a pre-selected temperature is indicative for at least the genus of a pathogen present in the sample, and
- the temperature dependence of said hybridization is indicative for at least the species of said pathogen.

The following example, references and sequence listing are provided to aid the understanding of the present invention, the true scope of which is set forth in the appended claims. It is understood that modifications can be made in the procedures set forth without departing from the spirit of the invention.

### Example

The following examples, references, sequence listing and figures are provided to aid the understanding of the present invention, the true scope of which is set forth in the appended claims. It is understood that modifications can be made in the procedures set forth without departing from the spirit of the invention.

### Samples

10³, 10², and 10 copies of plasmid DNA containing the ITS-1 region between the 18s rRNA and the 23sRNA genes from Enterococcus faecalis, Enterococcus faecium, Staphylococcus aureus, Staphylococcus epidermidis or Pseudomonas aeruginosa were analyzed partially in duplicate either in the absence or in the presence of 5 µg human genomic DNA background. An internal control plasmid was added to the completed mastermix (see above).

### Hardware / Software

A LightCycler™ instrument (Roche Diagnostics GmbH, Germany) was used. The commercially available instrument was modified in such a way that the rotor was adapted to hold 100 µl capillaries. The fluorimeter of said instrument was altered in such a way that it was composed of 4 instead of 3 photohybrids, and fluorescence emission could be detected at 610 nm, 640 nm, 670 nm and 705 nm.

### Reagents

All oligonucleotides mentioned herein were prepared by chemical synthesis. The reagents for attaching labels can be purchased from Roche Diagnostics GmbH (LightCycler Red 640 NHS Ester Cat.No. 2015161; LightCycler Red 705 Phosphoramidite Cat. No. 2157594; LightCycler Fluorescein (abbreviated 'F' in the following) CPG Cat. No. 3113906). The use of those reagents is described in Biochemica No. 1 (2001), p. 8-13. Cy5-NHS Ester can be obtained from Amersham upon request. LC-Red 610-NHS ester has an emission maximum at 610 nm and was synthesized according to standard protocols using a fluorescent dye as disclosed in US 5,750,409.

All reagents need to be checked for contamination by the organisms to be detected. Only reagents free of those organisms and the nucleic acids originating therefrom can lead to an optimum sensitivity.

FastStart polymerase and FastStart Master were generally used as recommended in the LightCycler-FastStart-DNA Master Hybridization Probes Kit (Roche Diagnostics GmbH Cat.No. 2239272)

Primers and probes directed against the ITS-1 region between the 16s and the 23s rDNA according to the following table were used

| | **Sequenz** | **Info** |
|---|---|---|
| **Enterococcus** | 5'-TAC-TTT-GTT-CAG-TTT- TGA-GAG-GT-3' SEQ.ID.NO: 1 | Forward Primer |
| | 5'-GCA-ATT-GAA-CTT-ATT-AAA-AAA-CTC-3' SEQ.ID.NO: 2 | Reverse Primer |
| | 5'-CTG-GAT-ATT-GAA-GTA-AAA-AGA-ATC-AAA-AC-X-3' SEQ.ID.NO: 3 | Fluos Probe I |
| | 5'-GAT-ATT-TGA-AGT-AAA-TGT-AAG-TAA-T-X-3' SEQ.ID.NO: 4 | Fluos Probe II |
| | 5'-LCRed₆₁₀-ACC-GAG-AAC-ACC-GCG-TTG-AAT-p-3' SEQ.ID.NO: 5 | Red 610 Probe |
| **Staphylococcus** | 5'-TGT ACA TTG AAA ACT AGA TAA GTA AG-3' SEQ.ID.NO: 6 | Forward Primer |
| | 5'-ACG CGT TAT TAA TCT TGT GAG T-3' SEQ.ID.NO: 7 | Reverse Primer |
| | 5'-CCG ACT GAA TAA AGA GTT TTA AA-X 3' SEQ.ID.NO: 8 | Fluos Probe I |
| | 5'-LCRED₆₄₀-CT TGA ATT CAT AAG AAA TAA TCG-3' SEQ.ID.NO: 9 | Red 640 Probe |
| **Pseudomonas** | 5'- TCT AAA ACA ATC GTC GAA AGC - 3' SEQ.ID.NO: 10 | Forward Primer |
| | 5'- CCG AAA ATT CGC GCT TGA AC - 3' SEQ.ID.NO: 11 | Reverse Primer |
| | 5'- GAA GTA AGA CTG AAT GAT CTC TT - X 3' SEQ.ID.NO: 12 | Fluos Probe I |
| | 5'- Cy-5-TCA CTG GTG ATC ATT CAA GTC AAG GT - 3' SEQ.ID.NO: 13 | Red 670 Probe |
| **Internal Control** | 5'-LCRED₇₀₅-CCA GTA GAA TGC CAA CC-3' SEQ.ID.NO. 14 | IC-Probe Red705 |

With these oligonucleotides, a reaction mix was prepared as follows:

| **Reagent** | **Used volume** | **Final concentration** |
|---|---|---|
| 10 x LC-FastStart DNA Master Hybridization Probe reagent | 10µl | Taq DNA Polymerase, reaction buffer 1mM MgCl₂, dNTPs |
| MgCl₂ (25 mM) | 10µl | 3.5mM |
| UNG (1U/µl) | 1µl | 2U |
| **Primers** | | |
| Primer Enterococ FP (20 pmol/µl) | 2.5 µl | 0.5µM |
| Primer Enterococ RP (20 pmol/µl) | 2.5 µl | 0.5µM |
| Primer StaphP30 (20 pmol/µl) | 2.5 µl | 0.5µM |
| Primer StaphP31 rev (20 pmol/µl) | 2.5 µl | 0.5µM |
| Primer Pseudo FP (20 pmol/µl) | 3.5 pl | 0.7µM |
| Primer Pseudo RP (20 pmol/µl) | 3.5 µl | 0.7µM |
| **Hybprobes** | | |
| Staphylococci : Staph Fluos (20 pmol/µl) | 1µl | 0.2µM |
| Staphylococci : Staph Red640 (20 pmol/µl) | 1µl | 0.2µM |
| Enterococci: Entero-Fluos I (20 pmol/µl) | 1µl | 0.2µM |
| Enterococci : Entero Fluos II (20 pmol/µ) | 1µl | 0.2µM |
| Enterococci: Entero Red610 (20 pmol/µl) | 1µl | 0.2µM |
| Pseudomonas : Pseudo Fluos (20 pmol/µl) | 1,5 µl | 0.2µM |
| Pseudomonas: Pseudo Red670 (20 pmol/µl) | 1,5 µl | 0.2µM |
| Internal Control : IC Red705 (20 pmol/µl) | 1µl | 0.2µM |
| Water (PCR grade, Roche) | Adjusted | |
| Target (Genomic bacterial DNA) | 5µl | 1000, 100 or 10 copies |
| Background human DNA (optional) | 5 µl | 5 µg/100 µl |
| Total volume | 100µl | |

### Method

A LightCycler-run was performed using the following thermocycling and melting temperature profile:

| | Cycles | time (sec) | Temp (°C) | Slope(°C) |
|---|---|---|---|---|
| Denaturation | 1 | 600 | 95°C | 20 |
| | | | | |
| Amplification | 10 | 10 | 95 | 20 |
| | | 25 | 60 | 20 |
| | | 50 | 72 | 20 |
| | | | | |
| Amplification | 35 | 10 | 95 | 20 |
| | | 25 | 50 | 20 |
| | | 10 | 72 | 20 |
| | | | | |
| Melting curve | 1 | 60 | 95 | 20 |
| | | 60 | 40 | 20 |
| | | | | |
| | | 0 | 80 | 0.1 |
| Cooling | 1 | 30 | 40 | 20 |

### Results

Results are summarized in the following table, indicating the Cp values (cycle number) at which, using the second derivative mode, a positive amplification signal could be detected.

| **Template** | **Copies** /**PCR** | **Quantification** CP hDNA | | no hDNA | |
|---|---|---|---|---|---|
| **E.faecalis** | 1000 | 23.96 | 24.035 | 24.34 | 24.43 |
| F3 | | 24.11 | | 24.52 | |
| | 100 | ---- | ---- | 27.17 | 27.15 |
| | | ---- | | 27.13 | |
| | 10 | ---- | ---- | 29 | 29.5 |
| | | ---- | | 30 | |
| **E.faecium** | 1000 | 23.7 | 23.82 | 24.74 | 24.81 |
| F3 | | 23.94 | | 24.88 | |
| | 100 | ---- | ---- | 26.16 | 26.96 |
| | | ---- | | 27.76 | |
| | 10 | ---- | ---- | 25.72 | 25.72 |
| | | ---- | | ---- | |
| **S.aureus** | 1000 | 21.03 | 21.045 | 21.12 | 21.335 |
| F4 | | 21.06 | | 21.55 | |
| | 100 | 22.15 | 21.625 | 24.12 | 23.335 |
| | | 21.1 | | 22.55 | |
| | 10 | ---- | 23.39 | 25.32 | 25.645 |
| | | 23.39 | | 25.97 | |
| **Sepidermidis** | 1000 | 22.73 | 22.73 | 25.73 | 25.725 |
| F4 | | ---- | | 25.72 | |
| | 100 | | ---- | ---- | ---- |
| | | ---- | | 26.71 | |
| | 10 | ---- | ---- | ---- | ---- |
| | | ---- | | **----** | |
| **P.aeroginosa** | 1000 | 22.22 | 22.23 | 22.26 | 22.275 |
| F5 | | 22.24 | | 22.29 | |
| | 100 | 24.31 | 23.705 | 25.19 | 25.19 |
| | | 23.1 | | 25.19 | |
| | 10 | 24.15 | 24.62 | 26.27 | 28.135 |
| | | 25.09 | | 30 | |

As can be seen from the table above, within this multiplex experiment, 10 copies of plasmid DNA containing either the ITS-1 region of either Enterococcus faecalis, Enterococcus faecium, Staphylococcus aureus or Pseudomonas aeruginosa could be detected, whereas for Staphylococcus epidermidis 100 copies were detectable.

In the presence of human backgound DNA, 10 copies of Staphylococcus aureus or Pseudomonas aeruginosa could be detected, whereas for Enterococcus faecalis, Enterococcus faecium and Staphylococcus epidermidis, 1000 copies were detectable.

Furthermore, and independent from the presence or absence of human background DNA, amplification of Enterococcus faecalis and Enterococcus faecium could be discriminated from each other in the 610 nm channel by melting curve analysis.

A Tm of 57,5°C was monitored for Enterococcus faecalis, whereas a Tm of 53,5 °C was monitored for Enterococcus faecium.

Similiarily, amplification of Staphylococcus aureus and Staphylococcus epidermidis could be discriminated from each other in the 640 nm channel by melting curve analysis due to a melting temp between the two melting peaks obtained.

For Staphylococcus aureus, a Tm of 54°C was obtained, whereas for Staphylococcus epidermidis, a TM of 45°C was obtained.

## Claims

1. Method for identification of a pathogenic organism from a predetermined group of pathogens, comprising
a) at least partially purifying nucleic acid from a clinical sample,
b) subjecting at least a first aliquot of said clinical specimen to at least one amplification and detection reaction in one reaction vessel comprising
ba) an amplification step using at least a first set of amplification primers capable of amplifying a pre-selected 16s/23s or 18s/26s spacer region from several or all members of said predetermined group of pathogens,
bb) a detection step using at least 2, 3 or multiple hybridization reagents, said reagents together being capable of specifically detecting a pre-selected 16s/23s or 18s/26s spacer region from all members of said group of pathogens, said detection step bb) comprising steps
bba) monitoring hybridization of each of said hybridization reagents at a pre-selected temperature, said hybridization being indicative for at least the genus of said pathogen present in the sample, and
bbb) monitoring temperature dependence of hybridization with said hybridization reagents and determining the melting temperature, said
temperature dependence being indicative for at least the species of said pathogen.
c) determining if a hybridization signal is occurring in step bba) whether a pathogenic organism of a certain genus is present in said sample, and determining from the temperature dependence of step bbb), which pathogenic species of said genus is present in said sample.

2. Method according to claim 1, wherein a first and a second aliquot each are subjected to an amplification and detection reaction independently from each other in two different reaction vessels,

3. Method according to claim 2, wherein a first, a second and a third aliquot each are subjected to an amplification and detection reaction independently from each other in two different reaction vessels.

4. Method according to claims 1-3, wherein an additional hybridization reagent is used for the detection of an internal control.

5. Method according to claim 2, wherein gram positive pathogenic organisms are exclusively identified in one amplification and detection reaction and gram negative pathogenic organisms are exclusively identified in another amplification and detection reaction.

6. Method according to claim 3 and 5, wherein fungal pathogens are exclusively identified in the third amplification and detection reaction.

7. Method according to claims 2-6, wherein each amplification step is performed with the same thermocycling profile.

## Patentansprüche

1. Verfahren zur Identifizierung eines pathogenen Organismus aus einer vorbestimmten Gruppe von Krankheitserregern, wobei man in dem Verfahren
a) Nukleinsäure wenigstens teilweise aus einer klinischen Probe aufreinigt,
b) wenigstens ein erstes Aliquot des klinischen Präparats wenigstens einer Amplifikations- und Nachweisreaktion in einem Reaktionsgefäß unterzieht, die
ba) einen Amplifikationsschritt unter Verwendung wenigstens eines ersten Satzes von Amplifikationsprimern, mit denen ein vorgewählter 16s/23s- oder 18s/26s-Spacer-Bereich aus mehreren oder allen Mitgliedern der vorbestimmten Gruppe von Krankheitserregern amplifiziert werden kann,
bb) einen Nachweisschritt unter Verwendung von wenigstens 2, 3 oder mehreren Hybridisierungsreagentien, wobei die Reagentien zusammen in der Lage sind, einen vorgewählten 16s/23s- oder 18s/26s-Spacer-Bereich aus allen Mitgliedern der Gruppe von Krankheitserregern spezifisch nachzuweisen, wobei der Nachweisschritt bb) die Schritte
bba) Überwachen der Hybridisierung eines jeden der Hybridisierungsreagentien bei einer vorgewählten Temperatur, wobei die Hybridisierung wenigstens das in der Probe vorliegende Genus des Krankheitserregers anzeigt, und
bbb) Überwachen der Temperaturabhängigkeit der Hybridisierung mit den Hybridisierungsreagentien und Bestimmen der Schmelztemperatur, wobei die Temperaturabhängigkeit wenigstens die Spezies des Krankheitserregers anzeigt,
umfasst,
c) bestimmt, ob bei Auftreten eines Hybridisierungssignals in Schritt bba) ein pathogener Organismus eines bestimmten Genus in der Probe vorliegt, und aus der Temperaturabhängigkeit aus Schritt bbb) bestimmt, welche pathogene Spezies des Genus in der Probe vorliegt.

2. Verfahren nach Anspruch 1, wobei ein erstes und ein zweites Aliquot jeweils einer Amplifikations- und Nachweisreaktion unabhängig voneinander in zwei verschiedenen Reaktionsgefäßen unterzogen werden.

3. Verfahren nach Anspruch 2, wobei ein erstes, ein zweites und ein drittes Aliquot jeweils einer Amplifikations- und Nachweisreaktion unabhängig voneinander in zwei verschiedenen Reaktionsgefäßen unterzogen werden.

4. Verfahren nach Anspruch 1-3, wobei ein zusätzliches Hybridisierungsreagens für den Nachweis einer internen Kontrolle verwendet wird.

5. Verfahren nach Anspruch 2, wobei ausschließlich grampositive pathogene Organismen in einer Amplifikations- und Nachweisreaktion und ausschließlich gramnegative pathogene Organismen in einer weiteren Amplifikations- und Nachweisreaktion identifiziert werden.

6. Verfahren nach Anspruch 3 und 5, wobei ausschließlich pilzliche Krankheitserreger in der dritten Amplifikations- und Nachweisreaktion identifiziert werden.

7. Verfahren nach Anspruch 2-6, wobei jeder Amplifikationsschritt mit dem gleichen Thermocycling-Profil ausgeführt wird.

## Revendications

1. Procédé pour l'identification d'un organisme pathogène à partir d'un groupe prédéterminé d'agents pathogènes, comprenant
a) la purification au moins partielle d'un acide nucléique d'un échantillon clinique,
b) la soumission d'au moins une première aliquote dudit spécimen clinique à au moins une réaction d'amplification et de détection dans un récipient de réaction comprenant
ba) une étape d'amplification utilisant au moins un premier jeu d'amorces d'amplification capables d'amplifier une région espaceur 16s/23s ou 18s/26s présélectionnée parmi plusieurs ou tous les membres dudit groupe prédéterminé d'agents pathogènes,
bb) une étape de détection utilisant au moins 2, 3 ou de multiples réactifs d'hybridation, lesdits réactifs étant capable ensemble de détecter spécifiquement une région espaceur 16s/23s ou 18s/26s présélectionnée parmi tous les membres dudit groupe d'agents pathogènes, ladite détection de l'étape bb) comprenant les étapes de
bba) surveillance de l'hybridation de chacun desdits réactifs d'hybridation à une température pré-sélectionnée, ladite hybridation étant indicatrice au moins du genre dudit agent pathogène présent dans l'échantillon,
et
bbb) surveillance de la dépendance de la température d'hybridation avec lesdits réactifs d'hybridation et détermination de la température de fusion, ladite dépendance de la température étant indicatrice au moins de l'espèce dudit agent pathogène.
c) la détermination si un signal d'hybridation survient à l'étape bba) si un organisme pathogène d'un certain genre est présent dans ledit échantillon, et la détermination à partir de la dépendance de la température de l'étape bbb), quelle espèce pathogène dudit genre est présente dans ledit échantillon.

2. Procédé selon la revendication 1, dans lequel une première et une deuxième aliquote sont soumises chacune à une réaction d'amplification et de détection de manière indépendante l'une de l'autre dans deux récipients de réactions différents.

3. Procédé selon la revendication 2, dans lequel une première, une deuxième et une troisième aliquote sont soumises chacune à une réaction d'amplification et de détection de manière indépendante l'une de l'autre dans deux récipients de réactions différents.

4. Procédé selon les revendications 1 à 3, dans lequel un réactif d'hybridation supplémentaire est utilisé pour la détection d'un étalon interne.

5. Procédé selon la revendication 2, dans lequel les organismes pathogènes gram positif sont identifiés exclusivement dans une réaction d'amplification et de détection et les organismes pathogènes gram négatif sont identifiés exclusivement dans une autre réaction d'amplification et de détection.

6. Procédé selon les revendications 3 et 5, dans lequel les agents pathogènes fongiques sont identifiés exclusivement dans la troisième réaction d'amplification et de détection.

7. Procédé selon les revendications 2 à 6, dans lequel chaque étape d'amplification est réalisée avec le même profil de thermocyclage.


### SEQUENCE LISTING

<110> Roche Diagnostics GmbH
   F. Hoffmann-La Roche AG
<120> Multiplex assay detection of pathogenic organisms
<130> 21719 EP
<140> EP03007458.7
   <141> 2003-04-04
<160> 14
<170> PatentIn Ver. 2.1
<210> 1
   <211> 23
   <212> DNA
   <213> Artificial Sequence
<220>
   <223> Description of Artificial Sequence:Forward Primer
<400> 1
   tactttgttc agttttgaga ggt 23
<210> 2
   <211> 24
   <212> DNA
   <213> Artificial Sequence
<220>
   <223> Description of Artificial Sequence:Reverse Primer
<400> 2
   gcaattgaac ttattaaaaa actc 24
<210> 3
   <211> 29
   <212> DNA
   <213> Artificial Sequence
<220>
   <223> Description of Artificial Sequence:Fluos Probe I
<400> 3
   ctggatattg aagtaaaaag aatcaaaac 29
<210> 4
   <211> 25
   <212> DNA
   <213> Artificial Sequence
<220>
   <223> Description of Artificial Sequence:Fluos Probe II
<400> 4
   gatatttgaa gtaaatgtaa gtaat 25
<210> 5
   <211> 21
   <212> DNA
   <213> Artificial Sequence
<220>
   <223> Description of Artificial Sequence:Red 610 Probe
<400> 5
   accgagaaca ccgcgttgaa t 21
<210> 6
   <211> 26
   <212> DNA
   <213> Artificial Sequence
<220>
   <223> Description of Artificial Sequence:Forward Primer
<400> 6
   tgtacattga aaactagata agtaag 26
<210> 7
   <211> 22
   <212> DNA
   <213> Artificial Sequence
<220>
   <223> Description of Artificial Sequence:Reverse Primer
<400> 7
   acgcgttatt aatcttgtga gt 22
<210> 8
   <211> 23
   <212> DNA
   <213> Artificial Sequence
<220>
   <223> Description of Artificial Sequence:Fluos Probe I
<400> 8
   ccgagtgaat aaagagtttt aaa 23
<210> 9
   <211> 24
<212> DNA
   <213> Artificial Sequence
<220>
   <223> Description of Artificial Sequence:Red 640 Probe
<400> 9
   gcttgaattc ataagaaata atcg 24
<210> 10
   <211> 21
   <212> DNA
   <213> Artificial Sequence
<220>
   <223> Description of Artificial Sequence:Forward Primer
<400> 10
   tctaaaacaa tcgtcgaaag c 21
<210> 11
   <211> 20
   <212> DNA
   <213> Artificial Sequence
<220>
   <223> Description of Artificial Sequence:Reverse Primer
<400> 11
   ccgaaaattc gcgcttgaac 20
<210> 12
   <211> 23
   <212> DNA
   <213> Artificial Sequence
<220>
   <223> Description of Artificial Sequence:Fluos Probe I
<400> 12
   gaagtaagac tgaatgatct ctt 23
<210> 13
   <211> 26
   <212> DNA
   <213> Artificial Sequence
<220>
   <223> Description of Artificial Sequence:Red 670 Probe
<400> 13
   tcactggtga tcattcaagt caaggt 26
<210> 14
   <211> 17
   <212> DNA
   <213> Artificial Sequence
<220>
   <223> Description of Artificial Sequence:IC-Probe Red705
<400> 14
   ccagtagaat gccaacc 17